# EUROPEAN PATENT SPECIFICATION

(11) **EP 1 880 667 B1**
(45) Date of publication and mention of the grant of the patent: **25.02.2015**
(21) Application number: 07112487.9
(22) Date of filing: 13.07.2007
(51) Int. Cl.: A61B 5/0476

(54) **Detection of focal epileptiform activity**
Erkennung fokussierter epileptiformer Handlungen
Détection d'activité épileptiforme focale

(30) Priority: 19.07.2006 US 458433
(43) Date of publication of application: 23.01.2008
(73) Proprietor: GENERAL ELECTRIC COMPANY, Schenectady, NY 12345 (US)
(72) Inventor: Sarkela, Mika, Espoo, Finland 02200 (FI)
(74) Representative: Illingworth-Law, William Illingworth

(56) References cited:
- EP-A- 1 820 444
- US-A- 4 421 122
- US-A1- 2005 007 091
- US-A1- 2006 009 709

## Description

### FIELD OF THE INVENTION

The present invention relates generally to the detection of focal epileptiform activity.

### BACKGROUND OF THE INVENTION

Electroencephalography (EEG) is a well-established method for assessing brain activity. When measurement electrodes are attached on the skin of the skull surface, the weak biopotential signals generated in brain cortex may be recorded and analyzed. The EEG has been in wide use for decades in basic research of the neural systems of the brain as well as in the clinical diagnosis of various central nervous system diseases and disorders. The EEG signal represents the sum of excitatory and inhibitory potentials of large numbers of cortical pyramidal neurons, which are organized in columns. Each EEG electrode senses the average activity of several thousands of cortical pyramidal neurons. The EEG signal is often divided into four different frequency bands: Delta (0.5 - 3.5 Hz), Theta (3.5 - 7.0 Hz), Alpha (7.0 - 13.0 Hz), and Beta (13.0 - 32.0 Hz). In an adult, Alpha waves are found during periods of wakefulness, and they may disappear entirely during sleep. Beta waves are recorded during periods of intense activation of the central nervous system. The lower frequency Theta and Delta waves reflect drowsiness and periods of deep sleep.

Different derangements of internal system homeostasis disturb the environment in which the brain operates, and therefore the function of the brain and the resulting EEG are disturbed. The EEG signal is a very sensitive measure of these neuronal derangements, which might be reflected in the EEG signal either as changes in membrane potentials or as changes in synaptic transmission. A change in synaptic transmission occurs whenever there is an imbalance between consumption and supply of energy in the brain. This means that the EEG signal serves as an early warning of a developing injury in the brain.

According to the present state of knowledge, the EEG signal is regarded as an effective tool for monitoring changes in the cerebral state of a patient. Diagnostically, the EEG is not specific, since many systemic disorders of the brain produce similar EEG manifestations. In Intensive Care Units (ICUs), an EEG signal may be of critical value, as it may differentiate between broad categories of psychogenic, epileptic, metabolic-toxic, encephalopatic and focal conditions.

Epilepsy is the most common neurological disorder, affecting about one per cent of the population during their lifetime. One proposed mechanism for the onset of an epileptic seizure is that neurons in a particular region of the brain become synchronized, leading to a reduction of EEG signal complexity in that area. The theory is proved correct by intracranial EEG recordings, cf. McSharry et al.: Comparison of Predictability of Epileptic Seizures by a Linear and Nonlinear Method, IEEE Transactions on Biomedical Engineering, vol. 50, No. 5, May 2003, pp. 628-633. However, when brain activity is recorded from the scalp, the measured signal is a composition originating from multiple sources, and methods indicative of the complexity of the signal show an increase during a seizure, cf. U.S. Patents 5,743,860 and 5,857,978.

At the most general level epileptic seizures can be classified into two subgroups: 1) generalized and 2) partial (focal) seizures. By definition, generalized seizures are seizures that start simultaneously from both hemispheres, whereas partial seizures start from one hemisphere.

The distinction between generalized and partial seizures is important for several reasons: the history, work up, and treatment of a patient with seizures are different depending on the subgroup. It is therefore important to observe if the seizure has any signatures of a focal onset. A well-defined aura, for example, indicates that the seizure began focally. During an aura, the rest of the brain is "watching" the part of the brain that has the seizure. In contrast, a generalized seizure does not have a well-defined aura. This is because consciousness requires one working cerebral hemisphere and a working brainstem. A generalized seizure in turn affects the entire brain at its onset.

Consequently, determination of an aura is very useful, and both the observer and the patient should be asked about the specific onset of the seizure. However, young children, for example, may be unable to verbalize an aura, and the aura or focal onset of a seizure may also be so brief that it may not be noticed before a secondary generalization of the seizure occurs. Thus, a focal seizure does not necessarily have an identifiable aura.

Since focal seizures are more likely to have underlying focal structural abnormalities, classification of focal vs. generalized seizures may affect the patient's work up. In addition, different anticonvulsants may be chosen depending on the classification.

ICU patients often suffer from brain disorders, which are originated by epilepsy or may evolve to epileptic seizures. However, identification of epileptic seizures of ICU patients is difficult, since the unconsciousness of the patients may be generated either by sedative drugs or by a brain disorder, such as an epileptic seizure. Furthermore, ICU patients often have non-convulsive forms of seizure activity, meaning that no simultaneous jerking motions exist. Therefore, EEG monitoring is the only method for detecting seizures of ICU patients. Discrimination of generalized and focal (or multifocal) epileptiform EEG activity is the most important categorization for the ICU patients, too, see for example Young GB, Mclachlan RS, Kreeft JH, Demelo JD. An Electroencephalographic Classification for Coma, The Canadian Journal of Neurological Sciences 1997; 24: 320-325.

Because multi-diseased ICU patients have several different etiologies for their brain disorders, their EEG may represent similar manifestations than EEG during epileptic seizures. These manifestations, i.e. EEG activity resembling epileptic EEG, are in this context termed epileptiforms. Epileptiform activity may be caused, for example, by different encephalopaties (septic, hepatic, renal, hypoxic-ischemic and toxic), central nervous system infections, brain tumors or injuries, subarachnoid or intracerebral hemorrhage, or ischemic strokes.

Epileptiform EEG activity caused by structural or functional lesions is typically focal. Radiological examinations, like magnetic resonance imaging (MRI) or computed tomography (CT), are typically performed to identify structural lesions. Imaging techniques like functional MRI and positron emission tomography (PET) may be used to identify functional lesions. However, imaging techniques are not 100% sensitive to neither type of lesions. Furthermore, functional imaging is cumbersome to perform. PET, for example, requires administration of short-lifetime radioactive nuclides to the patient. In a typical clinical environment, imaging can be performed maximally once per day, because of resource limitations.

Therefore, being a non-invasive, continuous and the most low-cost way to detect and follow up brain lesions, EEG-based monitoring offers many advantages as compared to radiological or imaging techniques.

EEG monitoring including at least one measurement channel on both hemispheres is needed for the purposes of the detection of focal epileptiform activity. In conventional EEG recording, cup or needle electrodes are positioned all over the head. The electrode attachment itself requires careful preparation of the skin and it is performed by specially educated nurses or technicians. However, in the so-called sub-hairline montage, see Bridgers SL, Ebersole JS. EEG outside the hairline: Detection of epileptiform activities, Neurology 1988; 38: 146-149, electrodes are attached to the non-hairy area of the head. The sub-hairline montage therefore offers a user-friendly method both for the comparison between the left and right hemispheres and for the comparison of anterior and posterior brain areas. The attachment of the electrodes does not require special skills and self-adhesive electrodes may be used, for example standard electrodes used for ECG recording. By increasing the number of EEG measurement channels, more detailed information about the precise origin of epileptiform activity may be obtained.

Some of the currently available EEG monitors have semi-automatic detectors for epileptiform activity. However, these monitors provide only binary information about epileptiform activity, i.e. they provide information only about the presence or absence of epileptiform activity. Due to the binary information utilized, the monitors are not efficient for detecting focal epileptiform activity or for localizing the focus of epileptiform activity. This drawback is due to the fact that focal epileptiform activity may be observable in large brain areas and may evolve even to the other hemisphere.

US 2005/007091 discloses specialized processing methods termed "complex ICA" or "complex independent component analysis" for determining independent signals from a mixture of signals, thus isolating a signal or signal component from the mixture of signals. Typically, the mixture of signals is received from a plurality of sensors such as EEG or Electrocardiogram sensors.

US-A-4 421 122 discloses generating a topographic display of information on brain electrical activity based on responses of electrical-activity transducers placed on the skull.

US 2006/0009709 relates to detection of encephalopathy based on EEG signal data obtained from a patient.

The present invention seeks to eliminate the above-mentioned drawback and to accomplish an EEG-based monitoring method with improved capability to detect and localize focal epileptiform activity.

### SUMMARY OF THE INVENTION

Aspects of the present invention are defined in the accompanying claims.

Embodiments of the present invention seek to provide a novel mechanism for detecting focal epileptiform activity. Embodiments of the present invention further seek to provide a mechanism that enables efficient localization of the focus of epileptiform activity based on time-domain brain wave signals, typically EEG signals, measured from a subject.

In an example of the present invention, a plurality of brain wave signals is measured from a subject. A measure indicative of the degree of epileptiform activity is determined for at least some of the signals, whereby a quantitative measure of the epileptiform activity is obtained for a plurality of the signals. A quantitative measure here refers to a measure which changes, in a monotonic manner, on a continuous scale according to the changes in the epileptiform activity. As discussed below, various parameters may be utilized as the quantitative measure. Based on the plurality of quantitative measures, an indication is provided of the presence of focal epileptiform activity. This involves that the system of the invention provides the user information from which the user may perceive whether or not focal epileptiform activity is present. The quantitative measures may, for example, be compared to each other to get an indication of the relative magnitudes of the signal-specific quantitative measures. If significant mutual differences are detected within the group of measures, focal epileptiform activity is detected.

Thus one embodiment of the invention is providing a method for detecting focal epileptiform activity. The method includes obtaining a plurality of brain wave signals from a subject and determining a signal-specific measure indicative of the degree of epileptiform activity for at least some of the plurality of brain wave signals, whereby a second plurality of signal-specific measures are obtained. The method further includes providing an indication of the presence of focal epileptiform activity based on the second plurality of signal-specific measures.

In a further embodiment of the invention, the occurrence of focal epileptiform activity is not only detected, but the focus of the epileptiform activity is localized based on the electrode positions associated with the brain wave signals.

Having the above-mentioned advantages of EEG-based monitoring methods, the mechanism of the invention provides an efficient and a low-cost screening tool by which patients requiring in-depth studies may be selected for imaging or radiological examinations. A further advantage of the invention is that the detection and localization algorithm does not require high computational power, which makes it suitable for various devices with limited computing power, such as ambulatory devices.

Another aspect of the invention is that of providing an apparatus for detecting focal epileptiform activity. The apparatus includes a measurement module configured to obtain a first plurality of brain wave signals from a subject and a first computing module configured to determine a signal-specific measure indicative of the degree of epileptiform activity for at least some of the first plurality of brain wave signals, whereby a second plurality of signal-specific measures are obtained. The apparatus further includes an indicator module configured to provide an indication of the presence of focal epileptiform activity based on the second plurality of signal-specific measures.

In a still further embodiment, the invention provides a computer program comprising a first program code portion configured to determine a signal-specific measure indicative of the degree of epileptiform activity for a plurality of brain wave signals, thereby to obtain a corresponding plurality of signal-specific measures and a second program code portion configured to provide an indication of the presence of focal epileptiform activity based on the second plurality of signal-specific measures. It is thus to be noted that since a conventional measurement device may be upgraded by a plug-in unit that includes software enabling the measurement device to detect focal epileptiform activity, the plug-in unit does not necessarily have to take part in the acquisition of the brain wave signal data.

Other features and advantages of the invention will become apparent by reference to the following detailed description and accompanying drawings.

### BRIEF DESCRIPTION OF THE DRAWINGS

In the following, the invention and its preferred embodiments are described more closely with reference to the examples shown in FIG. 1 to 6 in the appended drawings, wherein:
FIG. 1 is a flow diagram illustrating an embodiment of the method of the invention;
FIG. 2 illustrates one possible electrode configuration for measuring the brain wave signals from a subject;
FIG. 3 illustrates a further embodiment of the method of the invention;
FIG. 4a to 4d illustrate,respectively, four EEG channels measured from a patient;
FIG 4e illustrates the entropies of the wavelet coefficients calculated over a freguency band of 4 to 8 Hz for each of the EEG signals shown in FIGs. 4a to 4d;
FIG. 4f illustrates the standard deviation of the entropy values shown in FIG. 4e;
FIG. 5 illustrates one embodiment of the apparatus or system according to the invention; and
FIG. 6 illustrates the operational units of the control unit of FIG. 5 for detecting and localizing focal epileptiform activity.

### DETAILED DESCRIPTION OF THE INVENTION

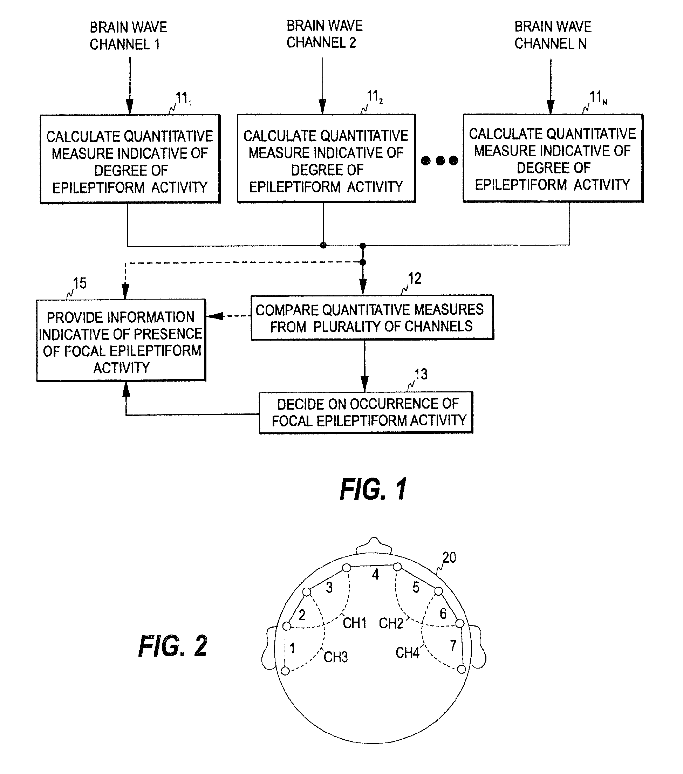

FIG. 1 is a flow diagram illustrating one embodiment of the method of the invention. In the present invention, N (N = 2, 3,...) brain wave signals are measured from a subject. As is common in the art, each incoming brain wave signal is sampled and the digitized signal samples are processed as sets of sequential signal samples representing finite time blocks or time windows, commonly termed "epochs". Here, each brain wave signal is also referred to as a channel, i.e. each brain wave signal is obtained through a corresponding channel.

Based on each brain wave signal, a measure indicative of the degree of epileptiform activity is determined (steps 11₁,..., 11_{N}), whereby N signal-specific values are obtained for the measure in each time window. As mentioned above, the measure here refers to a quantitative measure of epileptiform activity, which changes in a monotonic manner on a continuous scale according to the changes in the epileptiform activity.

Next, some or all of the N signal-specific values obtained during a time window are compared with each other at step 12 to examine whether there are significant differences between the signal-specific values obtained within the time window. If this is the case, the process decides at step 13 that focal epileptiform activity is present. If no significant differences are detected between the signal-specific values, the process decides that focal epileptiform activity is not present.

After step 13, the process provides the user of the system information about the results (step 15). As discussed below, this step may involve provision of diversified information based on which the user may perceive whether focal epileptiform activity is present or not.

In a typical embodiment of the invention, step 13 further includes the localization of the focus of the epileptiform activity when focal epileptiform activity is detected. This is performed based on the electrode positions of the individual signal channels: the electrode position(s) of the signal(s) with the strongest indication(s) of epileptiform activity represent(s) the focal area.

The quantitative measure determined for each brain wave signal in steps 11₁ to 11_{N} are derived as the entropy and as a normalized central moment, such as kurtosis, of the signal components on a desired subband of the brain wave signal. The subband may be derived by filtering the time-domain brain wave signals by a filter bank, for example. As discussed below, one possibility to implement the filter bank is to use a wavelet filter to decompose each brain wave signal into subbands so that at least one of the subbands corresponds to the epileptic waveforms of interest. In this case the entropy and kurtosis of the wavelet coefficients of the said at least one subband serve as the quantitative measure defined in steps 11₁ to 11_{N}.

The quantitative measures may also be derived, for example, from absolute or relative signal amplitudes detected during each successive time window. In order to improve the specificity of the method, the signal amplitudes may be examined on a certain frequency band. For example, the absolute EEG spike amplitudes or the EEG spike amplitudes relative to the background amplitudes may be determined, which occur on a frequency band on which epileptiform spikes occur. A spike here refers to a sharp transient with duration up to a certain maximum time length, such as 200 ms. Alternatively, the quantitative measure may be determined as the signal power on the said certain frequency band. For this, a Fourier transform may be employed to obtain the signal components on the said frequency band. The said certain frequency band may also be a band on which phasic epileptiform activity occurs. That is, the quantitative measure may also be determined as the absolute or relative signal amplitude or as the signal power on a band on which phasic epileptiform activity occurs.

The operations performed in the comparison step 12 may depend on the number of channels used. In case of only two channels (signals) the difference of the quantitative measures may be determined, whereas a measure of the amount of variability, such as the standard deviation, may be calculated if a greater number of channels is used.

In simplified embodiments of the invention, the system of the invention may only display the channel-specific quantitative measures and/or at least one difference of the said measures, whereby the user of the system may decide on the presence of focal epileptiform activity. In FIG. 1, these embodiments are illustrated by dashed arrows leading to step 15. Thus, in these embodiments the system does not make a decision on the presence of focal epileptiform activity, but the system only provides information based on which the user is able to make the said decision.

The measurement electrodes may be positioned to various positions around the scalp of a patient so that signals are obtained from both hemispheres. However, it is advantageous to employ an electrode configuration comprising one or more electrode pairs, so that the electrodes of each pair are positioned symmetrically onto opposite hemispheres. This is due to the fact that certain artifacts caused by eye movements, electrical activity of the heart and blood pulsation appear similarly in symmetrically positioned electrodes and thus tend to cancel out in the comparison step.

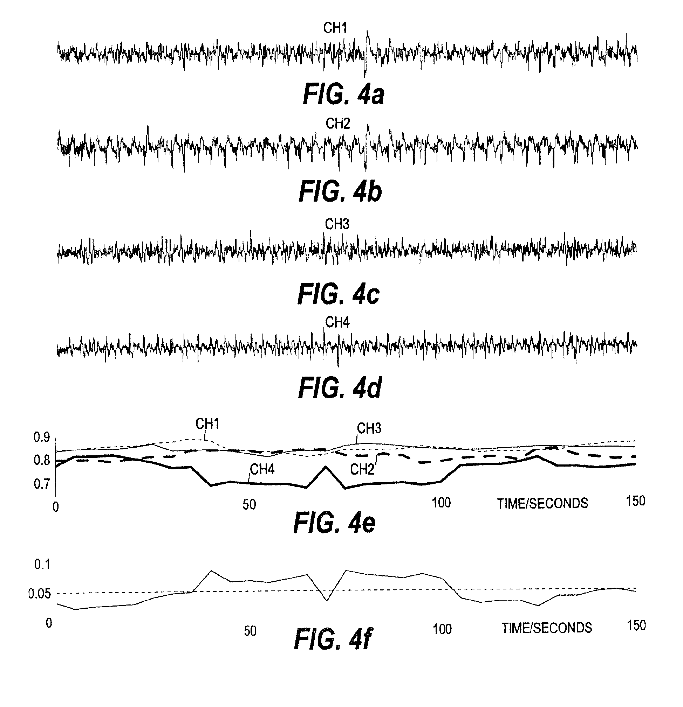

FIG. 2 illustrates one possible electrode configuration for measuring the brain wave channels from a subject. The figure shows a seven-channel circumferential bipolar configuration viewed from above the head 20 of a patient. This kind of electrode configuration is illustrated in the above-referred article of S.L. Bridgers and J.S. Ebersole, and it is advantageous in the sense that all the electrodes may be positioned below the hairline, which enables a quick and easy placement of the electrodes. The number of electrodes used for the N channels may vary, since two or more channels may have a common reference electrode. In the embodiment of FIG. 2, adjacent channels (numbered from 1 to 7) have a common electrode. The dashed curves provided with reference signs CH1 to CH4 relate to the example discussed below in connection with FIGs. 4a to 4f.

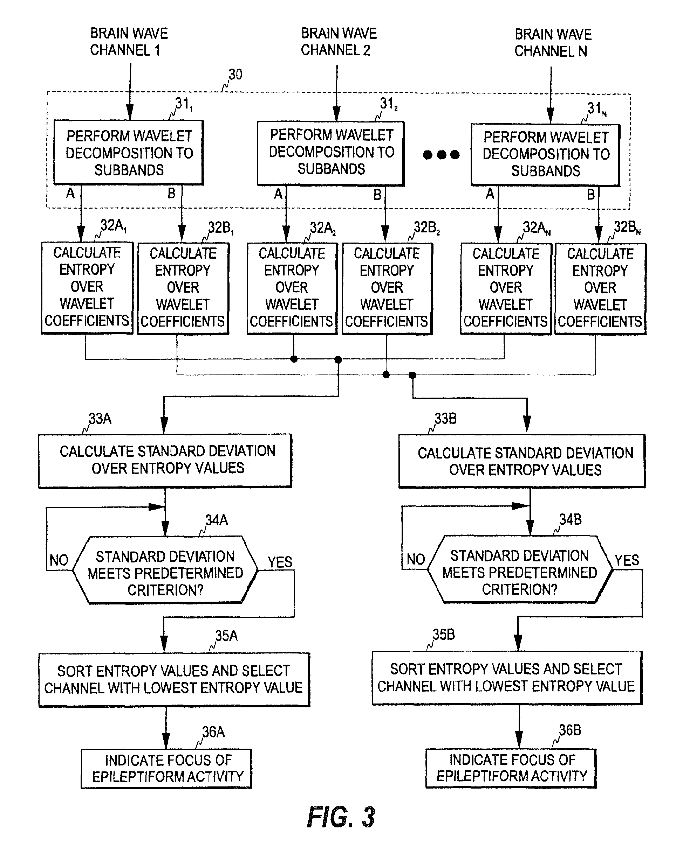

FIG. 3 illustrates an example of the invention, in which the signal-specific quantitative measure is the entropy or kurtosis of the wavelet coefficients of a subband of the frequency range on which epileptiform activity may appear.

In the embodiment of FIG. 3, a wavelet transform is utilized to separate two subbands from the frequency band on which epileptiform activity may appear, each subband corresponding to a specific type of epileptiform waveforms. Epileptiform EEG activity may include spiky waveforms. Although the frequency contents of the spikes may reach up to about 70 Hz, epileptiform EEG components are typically below 30 Hz. In the embodiment of FIG. 3, two bands are selected, which may contain epileptiform activity and a wavelet transform is employed to decompose each EEG signal obtained from a subject into the said two subbands (steps 31₁, to 31_{N}). The corresponding wavelet filter bank is denoted with reference numeral 30.

As a result of each decomposition process, two sets of wavelet coefficients are obtained. Each step 31ⱼ (j=1,..., N) thus outputs a first set of wavelet coefficients for the first subband (output A) and a second set of wavelet coefficients for the second subband (output B). The entropy of the respective wavelet coefficients is then determined at each step 32Aᵢ and 32Bᵢ (i=1,..., N), where A refers to the first subband and B to the second subband, i.e. in step 32Aᵢ the entropy of the wavelet coefficients of the first subband is determined for brain wave signal i and in step 32Bⱼ the entropy of the wavelet coefficients of the second subband is determined for brain wave signal j.

The standard deviation of the N simultaneous entropy values obtained for the first subband is then calculated at step 33A, whereas the standard deviation of the N simultaneous entropy values obtained for the second subband is calculated at step 33B. Both values of the standard deviation are then examined in separate processes, steps 34A and 34B, in order to detect whether the standard deviation meets at least one predetermined criterion set for the occurrence of focal epileptiform activity. Steps 34A and 34B thus serve as decision-making steps in which a decision is made on the occurrence of focal epileptiform activity specific to the subband in question, and they may include a comparison in which the calculated deviation value is compared with a predetermined threshold value. If the standard deviation exceeds the threshold value, the process decides that focal epileptiform activity is present in the signal data. If this is the case for any of the subbands, the process continues by sorting the entropy values of that subband and selects the signal with the lowest entropy value to represent the damaged brain area (steps 35A and 35B). The lowest value is selected since the entropy decreases during epileptiform activity. Based on the electrode position(s) corresponding to the selected value, the process may then indicate the focus of the epileptiform activity (steps 36A and 36B).

In one embodiment of the invention, the presence or absence of focal epileptiform activity is not indicated in each time window, but the process may make the decision based on the comparisons made in several successive time windows. The decision may be made, for example, based on a predetermined majority rule.

As discussed above, the N quantitative measures obtained for a subband may also be compared to each other pair-wise, and focal epileptiform activity may be detected if the difference (or another like variable) between the quantitative measures obtained from symmetrically positioned electrodes exceeds a predetermined threshold.

A method in which subband-specific entropies of wavelet coefficients are utilized to detect epileptiform activity is disclosed in Applicant's EP Patent Application EP 1 820 444.

As discussed therein, the wavelet transform may be employed to decompose the EEG signal into subbands so that at least one of the subbands corresponds to the epileptic waveforms of interest. Thus, in steps 31₁ to 31_{N} each EEG signal may be decomposed to one or more subbands of interest. The subbands that may be employed include subbands on which epileptiform spikes occur, such as 16 to 32 Hz and 32 to 64 Hz, and subbands on which phasic waves (triphasic, diphasic or monophasic) occur, such as 2 to 4 Hz and 4 to 8 Hz. In the embodiment of FIG. 3, the first subband may be, for example, a subband on which phasic waves occur, while the second subband may be a subband on which spikes occur. If subbands related to different types of epileptiform activity are employed simultaneously, the system of the invention thus detects when focal epileptiform activity of a specific type is present, and may thus indicate both the focus and the type of the epileptiform activity detected.

The mother wavelet to be used for the wavelet transform belongs preferably to the Daubechies (db) family or to the Symmlet (symm) family, since these families include wavelets that have a good match for actual epileptiform waveforms. Furthermore, it is advantageous to employ a basis function of a relatively low order, such as two or three, since the low order basis functions of a family represent epileptiform patterns better than the high order basis functions of the same family. This is because the basis functions become smoother and more oscillatory, i.e. less spiky, when the order increases. The specificity of the basis functions to spiky and phasic waveforms thus declines as the order increases.

As also discussed in the above-mentioned EP Patent Application, instead of the entropy of the wavelet coefficients, the kurtosis of the wavelet coefficients may also be used as the quantitative measure, i.e. instead of the entropy of the wavelet coefficients, the kurtosis of the wavelet coefficients may be determined in steps 32Aᵢ and 32Bᵢ. Furthermore, kurtosis, which is a normalized form of the fourth central moment, may also be replaced by a normalized form of a central moment of an order higher than four. If kurtosis is employed as the quantitative measure, the signal with the highest kurtosis value is selected to represent the damaged brain area in steps 35A and 35B (kurtosis increases during epileptiform activity).

The advantage of the use of the wavelet entropy or any of the above central moments is that the said parameters are specific to epileptiform activity. whereas the other quantitative measures mentioned above may be sensitive to other EEG features, such as normal EEG slowing, i.e. Delta and Theta activation, or to electromyographic (EMG) activity.

Next, the operation of the invention is illustrated with reference to the real-life examples illustrated in FIGs. 4a to 4f. FIGs. 4a to 4d illustrate, respectively, four EEG channels measured from a patient during epileptiform activity. The electrode arrangement used for the measurement corresponds to the arrangement shown in FIG. 2. The four channels, CH1 to CH4, are denoted by dashed curves in FIG. 2, each curve connecting the electrodes from between which the respective channel is measured. FIG 4e illustrates the entropies of the wavelet coefficients calculated over a freguency band of 4 to 8 Hz for each of the EEG signals of FIGs. 4a to 4d, i.e. the analysis seeks to detect phasic focal epileptiform activity. The mother wavelet used for the wavelet transform was Daubechies 3. FIG. 4f illustrates the standard deviation of the entropy values shown in FIG. 4e. FIG. 4f also shows a threshold (0.05) used in the comparison step. When focal epileptiform activity is present, the entropy of one of the channels (CH4) drops, which causes the standard deviation of the entropy values to rise above the threshold value.

The focal epileptiform activity is in this example a short focal disorder in the area of the right temporal lobe, a so-called periodical lateralized epileptiform discharge (PLED). As can be seen from FIG. 4f, the said seizure lasts about one minute.

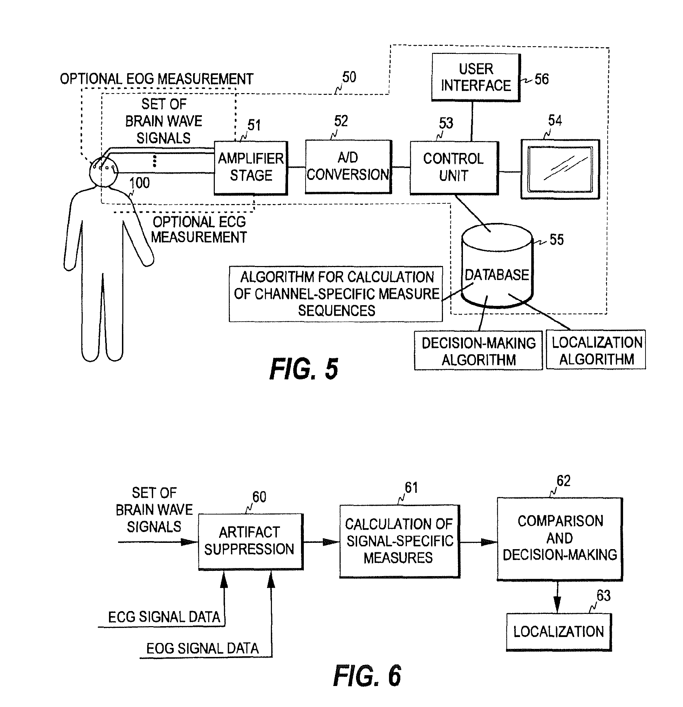

FIG. 5 illustrates one embodiment of the system according to the invention, assuming that the brain wave data acquired from a subject 100 is EEG signal data. At least two signals are measured by positioning the corresponding electrodes on different parts of the scalp of the subject. If only two signals are utilized, they are measured from opposite hemispheres.

The EEG signals obtained through the corresponding electrode arrangement are supplied to an amplifier stage 51, which amplifies the signals before they are sampled and converted into digitized format in an A/D converter 52. The digitized signals are supplied to a computer unit 53 which may comprise one or more processors.

The signal path of each EEG channel may also be provided with various pre-processing stages, such as filtering stages, which serve to remove non-idealities from the measured signal or otherwise improve the quality of the signal. In one embodiment of the invention, electrocardiographic (ECG) and/or electro-oculographic (EOG) signal data are measured simultaneously from the patient to remove ECG-based artefacts and/or eye movement artefacts from the EEG signal data obtained from the patient.

The signal processing operations may be implemented by dedicated (EEG channel-specific) processing units or by processing units common to at least two channels. Therefore, FIG. 5 only illustrates basic operations applied to the signals, without taking a position on the actual hardware implementation.

The control unit is provided with a database or memory unit 55 holding the digitized EEG signals. The actual recording of the EEG signals thus occurs in a conventional manner, i.e. a measurement device 50 including the above elements serves as a conventional EEG measurement device that acquires a plurality of EEG signals from the subject. However, if wavelet transforms are utilized in the control unit, the sampling frequency of the device may be set according to the requirements of the transform.

Additionally, the control unit is provided with the above-described algorithms for detecting focal epileptiform activity. As shown in FIG. 6, this embodiment of the control unit may include at least three successive operational entities: a calculation module 61 for deriving a sequence of the quantitative measure for each channel, a comparison module 62 for comparing the simultaneous quantitative measures of at least two channels, and a localization module 63 for localizing the focus of the epileptiform activity. Additionally, the control unit may comprise an artifact suppression module 60, which may receive EOG and ECG signal data in order to remove ECG-based artefacts and/or eye movement artefacts from the brain wave channels. As discussed above, the calculation module may include a filter bank which may carry out a Fourier transform or a wavelet transform, for example. As to the comparison module, it may not be necessary to process the measures of all channels, but the comparison module may indicate presence of focal epileptiform activity immediately upon detection of a significant difference between two measure values within entire set of values.

Although one control unit (processor) may perform the calculations needed, the processing of the brain wave signals may also be distributed among different processors (servers) within a network, such as a hospital LAN (local area network). For example, a conventional measurement device may record the brain wave signals and an external processor may be responsible for the detection of the focal epileptiform activity based on the signals. The term control unit here refers to any system, processor, circuit, or computing entity which is capable of carrying out the above operations based on brain wave signal data, so that either the system or its user may decide whether or not focal epileptiform activity is present.

The control unit may display the results on the screen of a monitor 54 connected to the control unit. This may be carried out in many ways using textual and/or graphical information about the presence of certain waveforms or patterns and their focus. For example, the monitor may display the skull and indicate the area corresponding to the focus for each type of epileptiform activity detected. However, in a simplified embodiment the apparatus may only indicate the channel-specific quantitative measures, in which case a physician may decide on the occurrence of focal epileptiform activity. The system further includes user interface means 56 through which the user may control the operation of the system.

As discussed above, the brain wave data may also be acquired through a standard MEG recording. The measurement device 50 may thus also serve as a conventional MEG measurement device, although a MEG measuring arrangement is far more expensive than an EEG measuring arrangement.

The software enabling a conventional EEG or MEG measurement device 50 to detect epileptiform waveforms may also be delivered separately to the measurement device, for example on a data carrier, such as a CD or a memory card, or through a telecommunications network. In other words, a conventional EEG or MEG measurement device may be upgraded by a plug-in unit that includes software enabling the measurement device to detect focal epileptiform activity based on the brain wave signals measured by the device from the subject. The software module thus determines, when run, the signal-specific quantitative measures and provides, based on the measures, an indication of the presence of epileptiform activity.

Although the invention was described above with reference to the examples shown in the appended drawings, it is obvious that the invention is not limited to these, but may be modified by those skilled in the art without departing from the scope of the invention. For example, the quantitative measure may be formed by combining two or more measures.

## Claims

1. A method for detecting focal epileptiform activity, the method comprising the steps of:
obtaining a plurality of brain wave signals (CH1-CH4) from a subject (100);
decomposing (30) the brain wave signals into subbands so that at least one of the subbands corresponds to the epileptic waveforms of interest;
determining (32A_{I} -32B_{N}) a signal-specific quantitative measure indicative of the degree of epileptiform activity for at least some of the plurality of brain wave signals, whereby a plurality of signal-specific measures are obtained, a first measure being indicative of the entropy of a subband and a second measure being indicative of the fourth or higher normalized central moment of a subband; and
providing (12, 13, 15) an indication of the presence of focal epileptiform activity based on the plurality of signal-specific measures, wherein the providing step includes a sub-step of comparing (12) at least some of the plurality of signal-specific measures with each other.

2. A method according to claim 1, further comprising the steps of:
positioning a plurality of electrodes onto predetermined areas of the scalp (20) of the subject to obtain the plurality of brain wave signals through the plurality of electrodes,
whereby each signal-specific measure is associated with a predetermined area of the scalp; and
localizing (33A-36A; 33B-36B) the focus of the epileptiform activity based on the predetermined areas and the plurality of signal-specific measures.

3. A method according to claim 2, wherein the localizing step includes the substeps of:
sorting the plurality of signal-specific measures to obtain a sorted order of the signal-specific measures;
selecting one of the outermost signal-specific measures in the sorted order; and
finding the predetermined area associated with the selected signal-specific measure.

4. A method according to claim 1, wherein the determining step includes a sub-step of employing a filter (30) to decompose each brain wave signal into at least one predetermined subband.

5. A method according to claim 1, wherein the determining step includes the substeps of:
defining spike amplitudes for the at least some of the plurality of brain wave signals (CH1-CH4); and
determining the plurality of signal-specific measures based on the spike amplitudes.

6. A method according to claim 1, wherein the comparing sub-step includes calculating a measure indicative of the amount of variability in the plurality of signal-specific measures.

7. A method according to any one of the preceding claims, wherein the providing step includes indicating (36A, 36B) the focus of the epileptiform activity.

8. An apparatus for detecting focal epileptiform activity, the apparatus comprising:
measurement means (50) for obtaining a plurality of brain wave signals (CH1-CH4) from a subject (100);
a filter (30) to decompose the brain wave signals into subbands so that at least one of the subbands corresponds to the epileptic waveforms of interest;
calculation means (53; 61) for determining a signal-specific quantitative measure indicative of the degree of epileptiform activity for at least some of the plurality of brain wave signals, whereby a plurality of signal-specific measures are obtained , a first measure being indicative of the entropy of a subband and a second measure being indicative of the fourth or higher normalized central moment of a subband; and
indicator means (53, 54), responsive to the calculation means, for providing an indication of the presence of focal epileptiform activity, the indicator means comprising comparison means (62) for comparing at least some of the plurality of signal-specific measures with each other.

9. An apparatus according to claim 8, further comprising:
a plurality of electrodes to be positioned onto predetermined areas of the scalp (20) of the subject (100) for obtaining the plurality of brain wave signals through the plurality of electrodes, whereby each signal-specific measure is associated with a predetermined area of the scalp; and
localization means (63) for localizing the focus of the epileptiform activity based on the predetermined areas and the plurality of signal-specific measures.

10. An apparatus according to claim 9, wherein the localization means (63) comprise:
sorting means for sorting the plurality of signal-specific measures to obtain a sorted order of the signal-specific measures;
selection means for selecting one of the outermost signal-specific measures in the sorted order; and
association means for finding the predetermined area associated with the selected signal-specific measure.

11. An apparatus according to claim 8, wherein the calculation means comprise a filter bank (30) for decomposing the at least some of the plurality of brain wave signals into at least one predetermined subband.

12. An apparatus according to claim 8, wherein the calculation means (53; 61) are configured to define spike amplitudes for the at least some of the plurality of brain wave signals and determine the plurality of signal-specific measures based on the spike amplitudes.

13. An apparatus according to claim 8, wherein the comparison means (62) are configured to calculate a measure indicative of the amount of variability in the plurality of signal-specific measures.

14. A computer program product for detecting focal epileptiform activity, the computer program product comprising:
a first program code portion configured to decompose the brain wave signals into subbands so that at least one of the subbands corresponds to the epileptic waveforms of interest and to determine a signal-specific quantitative measure indicative of the degree of epileptiform activity for a plurality of brain wave signals, thereby to obtain a corresponding plurality of signal-specific measures, a first measure being indicative of the entropy of a subband and a second measure being indicative of the fourth or higher normalized central moment of a subband; and
a second program code portion configured to provide an indication of the presence of focal epileptiform activity based on the plurality of signal-specific measures, including a sub-step of comparing at least some of the plurality of signal-specific measures with each other.

## Patentansprüche

1. Verfahren zum Nachweisen von fokaler epileptiformer Aktivität, wobei das Verfahren die folgenden Schritte beinhaltet:
Einholen von mehreren Gehirnwellensignalen (CH1-CH4) von einem Subjekt (100);
Zerlegen (30) der Gehirnwellensignale in Subbänder, so dass wenigstens eines der Subbänder den epileptischen Wellenformen von Interesse entspricht;
Ermitteln (32A_{I}-32B_{N}) einer signalspezifischen quantitativen Messgröße, die den Grad an epileptiformer Aktivität für wenigstens einige der mehreren Gehirnwellensignale anzeigt, so dass mehrere signalspezifische Messgrößen erhalten werden, wobei eine erste Messgröße die Entropie eines Subbandes anzeigt und eine zweite Messgröße das vierte oder höhere normalisierte zentrale Moment eines Subbandes anzeigt; und
Bereitstellen (12, 13, 15) einer Anzeige der Anwesenheit von fokaler epileptiformer Aktivität auf der Basis der mehreren signalspezifischen Messgrößen, wobei der Bereitstellungsschritt einen Unterschritt des Vergleichens (12) von wenigstens einigen der mehreren signalspezifischen Messgrößen miteinander beinhaltet.

2. Verfahren nach Anspruch 1, das ferner die folgenden Schritte beinhaltet:
Positionieren mehrerer Elektroden auf vorbestimmten Bereichen der Kopfhaut (20) des Subjekts, um die mehreren Gehirnwellensignale durch die mehreren Elektroden zu erhalten, so dass jede signalspezifische Messgröße mit einem vorbestimmten Bereich der Kopfhaut assoziiert wird; und
Lokalisieren (33A-36A; 33B-36B) des Fokus der epileptiformen Aktivität auf der Basis der vorbestimmten Bereiche und der mehreren signalspezifischen Messgrößen.

3. Verfahren nach Anspruch 2, wobei der Lokalisierungsschritt die folgenden Unterschritte beinhaltet:
Sortieren der mehreren signalspezifischen Messgrößen zum Einholen einer sortierten Reihenfolge der signalspezifischen Messgrößen;
Auswählen von einer der äußersten signalspezifischen Messgrößen in der sortierten Reihenfolge; und
Finden des mit der gewählten signalspezifischen Messgröße assoziierten vorbestimmten Bereichs.

4. Verfahren nach Anspruch 1, wobei der Ermittlungsschritt einen Unterschritt des Anwendens eines Filters (30) zum Zerlegen jedes Gehirnwellensignals in wenigstens ein vorbestimmtes Subband beinhaltet.

5. Verfahren nach Anspruch 1, wobei der Ermittlungsschritt die folgenden Unterschritte beinhaltet:
Definieren von Spitzenamplituden für die wenigstens einigen der mehreren Gehirnwellensignale (CH1-CH4); und
Ermitteln der mehreren signalspezifischen Messgrößen auf der Basis der Spitzenamplituden.

6. Verfahren nach Anspruch 1, wobei der Vergleichsunterschritt das Berechnen einer Messgröße beinhaltet, die die Menge an Variabilität in den mehreren signalspezifischen Messgrößen anzeigt.

7. Verfahren nach einem der vorherigen Ansprüche, wobei der Bereitstellschritt das Anzeigen (36A, 36B) der epileptiformen Aktivität beinhaltet.

8. Vorrichtung zum Nachweisen von fokaler epileptiformer Aktivität, wobei die Vorrichtung Folgendes umfasst:
Messmittel (50) zum Einholen von mehreren Gehirnwellensignalen (CH1-CH4) von einem Subjekt (100);
ein Filter (30) zum Zerlegen der Gehirnwellensignale in Subbänder, so dass wenigstens eines der Subbänder den epileptischen Wellenformen von Interesse entspricht; Rechenmittel (53; 61) zum Bestimmen eines spezifischen quantitativen Messsignals, das den Grad an epileptiformer Aktivität für wenigstens einige der mehreren Gehirnwellensignale anzeigt, so dass mehrere signalspezifische Messgrößen erhalten werden, wobei eine erste Messgröße die Entropie eines Subbandes anzeigt und eine zweite Messgröße das vierte oder höhere normalisierte zentrale Moment eines Subbandes anzeigt; und
Anzeigemittel (53, 54), die auf die Rechenmittel ansprechen, zum Geben einer Anzeige der Anwesenheit von fokaler epileptiformer Aktivität, wobei das Anzeigemittel Vergleichsmittel (62) zum Vergleichen von wenigstens einigen der mehreren signalspezifischen Messgrößen miteinander umfasst.

9. Vorrichtung nach Anspruch 8, die ferner Folgendes umfasst:
mehrere Elektroden zum Positionieren auf vorbestimmten Bereichen der Kopfhaut (20) des Subjekts (100) zum Einholen der mehreren Gehirnwellensignale durch die mehreren Elektroden, so dass jede signalspezifische Messgröße mit einem vorbestimmten Bereich der Kopfhaut assoziiert wird; und
Lokalisierungsmittel (63) zum Lokalisieren des Fokus der epileptiformen Aktivität auf der Basis der vorbestimmten Bereiche und der mehreren signalspezifischen Messgrößen.

10. Vorrichtung nach Anspruch 9, wobei die Lokalisierungsmittel (63) Folgendes umfassen:
Sortiermittel zum Sortieren der mehreren signalspezifischen Messgrößen zum Einholen einer sortierten Reihenfolge der signalspezifischen Messgrößen;
Auswahlmittel zum Auswählen von einer der äußersten signalspezifischen Messgrößen in der sortierten Reihenfolge; und
Assoziierungsmittel zum Finden des mit der ausgewählten signalspezifischen Messgröße assoziierten vorbestimmten Bereichs.

11. Vorrichtung nach Anspruch 8, wobei die Rechenmittel eine Filterbank (30) zum Zerlegen der wenigstens einigen der mehreren Gehirnwellensignale in wenigstens ein vorbestimmtes Subband umfassen.

12. Vorrichtung nach Anspruch 8, wobei die Rechenmittel (53; 61) zum Definieren von Spitzenamplituden für die wenigstens einigen der mehreren Gehirnwellensignale und zum Ermitteln der mehreren signalspezifischen Messgrößen auf der Basis der Spitzenamplituden konfiguriert sind.

13. Vorrichtung nach Anspruch 8, wobei die Vergleichsmittel (62) zum Berechnen einer Messgröße konfiguriert sind, die die Menge an Variabilität in den mehreren signalspezifischen Messgrößen anzeigt.

14. Computerprogrammprodukt zum Nachweisen von fokaler epileptiformer Aktivität, wobei das Computerprogrammprodukt Folgendes umfasst:
einen ersten Programmcodeteil, konfiguriert zum Zerlegen der Gehirnwellensignale in Subbänder ist, so dass wenigstens eines der Subbänder den epileptischen Wellenformen von Interesse entspricht, und zum Ermitteln einer signalspezifischen quantitativen Messgröße, die den Grad an epileptiformer Aktivität für mehrere Gehirnwellensignale anzeigt, um dadurch eine entsprechende Mehrzahl von signalspezifischen Messgrößen zu erhalten, wobei eine erste Messgröße die Entropie eines Subbandes anzeigt und eine zweite Messgröße das vierte oder höhere normalisierte zentrale Moment eines Subbandes anzeigt; und
einen zweiten Programmcodeteil, der zum Geben einer Anzeige der Anwesenheit von fokaler epileptiformer Aktivität auf der Basis der mehreren signalspezifischen Messgrößen konfiguriert ist, einschließlich eines Unterschrittes des Vergleichens von wenigstens einigen der mehreren signalspezifischen Messgrößen miteinander.

## Revendications

1. Procédé de détection d'une activité épileptiforme focale, le procédé comprenant les étapes consistant à :
obtenir une pluralité de signaux d'ondes cérébrales (CH1-CH4) provenant d'un sujet (100) ;
décomposer (30) les signaux d'ondes cérébrales en sous-bandes de sorte qu'au moins l'une des sous-bandes corresponde aux formes d'ondes épileptiques d'intérêt ;
déterminer (32A_{I}-32B_{N}) une mesure quantitative spécifique au signal indicative du degré d'activité épileptiforme pour au moins certains de la pluralité de signaux d'ondes cérébrales, de manière à obtenir une pluralité de mesures spécifiques aux signaux,
une première mesure étant indicative de l'entropie d'une sous-bande et une seconde mesure étant indicative du quatrième moment central normalisé ou plus d'une sous-bande ; et
fournir (12, 13, 15) une indication de la présence d'une activité épileptiforme focale sur la base de la pluralité de mesures spécifiques aux signaux, dans lequel l'étape de fourniture comprend une sous-étape de comparaison (12) d'au moins certaines de la pluralité de mesures spécifiques aux signaux l'une avec l'autre.

2. Procédé selon la revendication 1, comprenant en outre les étapes consistant à :
positionner une pluralité d'électrodes sur des zones prédéterminées du cuir chevelu (20) du sujet pour obtenir la pluralité de signaux d'ondes cérébrales à travers la pluralité d'électrodes, de sorte que chaque mesure spécifique au signal soit associée à une zone prédéterminée du cuir chevelu ; et
localiser (33A-36A ; 33B-36B) le foyer de l'activité épileptiforme sur la base des zones prédéterminées et de la pluralité de mesures spécifiques aux signaux.

3. Procédé selon la revendication 2, dans lequel l'étape de localisation comprend les sous-étapes consistant à :
trier la pluralité de mesures spécifiques aux signaux pour obtenir un ordre trié des mesures spécifiques aux signaux ;
choisir l'une des mesures spécifiques aux signaux les plus externes dans l'ordre trié ; et
trouver la zone prédéterminée associée à la mesure spécifique au signal choisie.

4. Procédé selon la revendication 1, dans lequel l'étape de détermination comprend une sous-étape d'utilisation d'un filtre (30) pour décomposer chaque signal d'ondes cérébrales en au moins une sous-bande prédéterminée.

5. Procédé selon la revendication 1, dans lequel l'étape de détermination comprend les sous-étapes consistant à :
définir des amplitudes de pic pour les au moins certains de la pluralité de signaux d'ondes cérébrales (CH1-CH4) ; et
déterminer la pluralité de mesures spécifiques aux signaux sur la base des amplitudes de pic.

6. Procédé selon la revendication 1, dans lequel la sous-étape de comparaison comprend le calcul d'une mesure indicative de la quantité de variabilité dans la pluralité de mesures spécifiques aux signaux.

7. Procédé selon l'une quelconque des revendications précédentes, dans lequel l'étape de fourniture comprend l'indication (36A, 36B) du foyer de l'activité épileptiforme.

8. Appareil de détection de l'activité épileptiforme focale, l'appareil comprenant :
des moyens de mesure (50) pour obtenir une pluralité de signaux d'ondes cérébrales (CH1-CH4) d'un sujet (100) ;
un filtre (30) pour décomposer les signaux d'ondes cérébrales en sous-bandes de sorte qu'au moins l'une des sous-bandes corresponde aux formes d'ondes épileptiques d'intérêt ;
des moyens de calcul (53 ; 61) pour déterminer une mesure quantitative spécifique au signal indicative du degré d'activité épileptiforme pour au moins certains de la pluralité de signaux d'ondes cérébrales, de manière à obtenir une pluralité de mesures spécifiques aux signaux, une première mesure étant indicative de l'entropie d'une sous-bande et une seconde mesure étant indicative du quatrième moment central normalisé ou plus d'une sous-bande ; et
des moyens indicateurs (53, 54) sensibles aux moyens de calcul pour fournir une indication de la présence d'une activité épileptiforme focale, les moyens indicateurs comprenant des moyens de comparaison (62) pour comparer au moins certaines de la pluralité de mesures spécifiques aux signaux l'une avec l'autre.

9. Appareil selon la revendication 8, comprenant en outre :
une pluralité d'électrodes à positionner sur des zones prédéterminées du cuir chevelu (20) du sujet (100) pour obtenir la pluralité de signaux d'ondes cérébrales à travers la pluralité d'électrodes, de sorte que chaque mesure spécifique au signal soit associée à une zone prédéterminée du cuir chevelu ; et
des moyens de localisation (63) pour localiser le foyer de l'activité épileptiforme sur la base des zones prédéterminées et de la pluralité de mesures spécifiques aux signaux.

10. Appareil selon la revendication 9, dans lequel les moyens de localisation (63) comprennent :
des moyens de triage pour trier la pluralité de mesures spécifiques aux signaux afin d'obtenir un ordre trié des mesures spécifiques aux signaux ;
des moyens de sélection pour choisir l'une des mesures spécifiques aux signaux les plus externes dans l'ordre trié ; et
des moyens d'association pour trouver la zone prédéterminée associée à la mesure spécifique au signal choisie.

11. Appareil selon la revendication 8, dans lequel les moyens de calcul comprennent un bloc de filtres (30) pour décomposer les au moins certains de la pluralité de signaux d'ondes cérébrales en au moins une sous-bande prédéterminée.

12. Appareil selon la revendication 8, dans lequel les moyens de calcul (53 ; 61) sont configurés pour définir des amplitudes de pic pour les au moins certains de la pluralité de signaux d'ondes cérébrales et déterminer la pluralité de mesures spécifiques aux signaux sur la base des amplitudes de pic.

13. Appareil selon la revendication 8, dans lequel les moyens de comparaison (62) sont configurés pour calculer une mesure indicative de la quantité de variabilité dans la pluralité de mesures spécifiques aux signaux.

14. Produit de programmation informatique pour détecter une activité épileptiforme focale, le produit de programmation informatique comprenant :
une première portion de code de programmation configurée pour décomposer les signaux d'ondes cérébrales en sous-bandes de sorte qu'au moins l'une des sous-bandes corresponde aux formes d'ondes épileptiques d'intérêt et pour déterminer une mesure quantitative spécifique au signal indicative du degré d'activité épileptiforme pour une pluralité de signaux d'ondes cérébrales, de manière à obtenir une pluralité correspondante de mesures spécifiques aux signaux, une première mesure étant indicative de l'entropie d'une sous-bande et une seconde mesure étant indicative du quatrième moment central normalisé ou plus d'une sous-bande ; et
une seconde portion de code de programmation configurée pour fournir une indication de la présence d'une activité épileptiforme focale sur la base de la pluralité de mesures spécifiques aux signaux, comprenant une sous-étape de comparaison d'au moins certaines de la pluralité de mesures spécifiques aux signaux l'une avec l'autre.
